(19) Europäisches Patentamt
European Patent Office
Office européen des brevets

(11) EP 2 846 293 A2

(12) **EUROPEAN PATENT APPLICATION**

(43) Date of publication:
11.03.2015 Bulletin 2015/11

(51) Int Cl.:
**G06K 9/00** (2006.01)

(21) Application number: 14192125.4

(22) Date of filing: 19.03.2008

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(30) Priority: **19.03.2007 GB 0705223**

(62) Document number(s) of the earlier application(s) in accordance with Art. 76 EPC:
**08718814.0 / 2 137 672**

(27) Previously filed application:
**19.03.2008 PCT/GB2008/000977**

(71) Applicant: **Texrad Limited**
**Chilcompton, Somerset BA3 4HP (GB)**

(72) Inventors:
- **Ganeshan, Balaji**
  **Hove, Sussex BN3 1RJ (GB)**
- **Miles, Kenneth**
  **Kingston, Sussex BN7 3PB (GB)**
- **Young, Robert**
  **Peacehaven, Sussex BN10 7QQ (GB)**
- **Chatwin, Christopher**
  **Hove, Sussex BN3 2WL (GB)**

(74) Representative: **Gray, Peter John Bracey**
**THOMPSON GRAY LLP**
**Central Court**
**25 Southampton Buildings**
**London**
**WC2A 1AL (GB)**

(54) **METHOD, APPARATUS AND COMPUTER PROGRAM FOR ANALYSING MEDICAL IMAGE DATA**

(57) Medical image data is analysed to produce a biomarker. The data is filtered with a plurality of band-pass filters each having a different bandwidth. A texture parameter is then determined from the filtered data from each filter and the biomarker is determined as at a ratio of the texture parameters. When the biomarker is obtained from a CT image of a liver, it can be predictive of poor survival, disease extent and liver physiology of a patient following resection of colorectal cancer. When obtained from a mammographic image, the biomarker can be indicative of cancer invasion and receptor status within mammographic abnormalities. When obtained from a CT image of a lung nodule, the biomarker can be predictive of tumour stage (or grading) and tumour metabolism of a patient with non-small cell lung carcinoma (lung cancer). When obtained from an MRI image of the brain, the biomarker can be indicative of schizophrenia and/or other brain disorders.

Fig. 6

**Description**

FIELD OF THE INVENTION

The invention relates to image analysis for assisting medical diagnosis or prognosis, and in particular to the analysis of textural data.

BACKGROUND TO THE INVENTION

Images of parts of the body are commonly used to assisting with medical diagnosis and prognosis. Images include X-ray images, in particular computed tomography (CT) and mammographic images, and magnetic resonance imaging (MRI) images. Visual inspection of such images can be very effective. However, increasingly, image processing techniques are being employed to enhance the usefulness of these images and the accuracy of diagnosis and prognosis.

For example, colorectal cancer patients entering surveillance programs do not represent a uniform population of equal risk of recurrence. It is desirable to identify predictive factors that are linked to outcomes in order to allow modification of surveillance strategies for sub-groups of patients. Of particular interest is the use of imaging techniques for this purpose.

Some previous studies into the use of CT images have used texture analysis and have been based on segmentation and classification of visible focal lesions into benign and malignant, and on recognition of different organs using wavelet techniques and artificial neural network based decision algorithms. See, for example, "Automatic segmentation and classification of diffused liver diseases using wavelet based texture analysis and neural network", Mala K, Sadasivam V., INDICON, 2005 Annual IEEE 2005; 216-219.

However it is more complex and challenging to distinguish diagnostic patient groups from visually normal areas of the liver of patients following resection of colorectal cancer. Previous studies have shown potential for hepatic CT texture to differ between normal livers and apparently normal areas of tissue within livers bearing tumours and may reflect hepatic vascularity. See, for example, "Texture analysis of CT-images for early detection of liver malignancy", Mir A.H., Hanmandlu M., Tandon S.N., Biomed Sci Instrum. 1995; 31:213-7.

As another example, mammographic breast screening has resulted in a dramatic increase in the diagnosis of ductal carcinoma in situ (DCIS) among all mammographically detected cancers. The detection of DCIS on core biopsy is quite frequently followed by evidence of invasion within the final excision specimen and results in the need of a second operative procedure which includes axillary lymphadenectomy. Therefore an effective way of estimating the likelihood of an invasive focus preoperatively in patients diagnosed with DCIS would assist in better treatment planning and optimal use of sentinel node biopsy or axillary lymphadenectomy.

In mammography, computer-assisted diagnosis (CAD) is employed for automated detection of micro-calcification clusters and classification of masses as benign or malignant. Computer based mammographic image texture analysis includes density variations within masses, two-step scheme of pixel-level detection, region-level classification, automated feature-based microcalcification extraction, gradient and flow-based texture analysis. See, for example, "An automatic method to discriminate malignant masses from normal tissue in digital mammograms", Brake G.M., Karssemeijer N., Hendriks J.H., Phys. Med. Biol. 2000; 45, 2843-2857. The use of computer analysis to characterise rather than detect mammographic abnormalities is more challenging and less well developed.

The invention seeks to improve upon such techniques.

SUMMARY OF THE INVENTION

According to a first aspect of the invention there is provided a method of analysing medical image data to produce a biomarker, comprising:

- filtering the data with a plurality of band-pass filters each having a different bandwidth;
- determining a texture parameter from the filtered data from each filter;
- determining at least one ratio of the texture parameters for use as a biomarker.

According to a second aspect of the invention there is provided an apparatus for analysing medical image data to produce a biomarker, comprising:

- means for filtering medical image data with a plurality of band-pass filters each having a different bandwidth;
- means for determining a texture parameter from the filtered data from each filter;
- means for determining at least one ratio of the texture parameters for use as a biomarker.

The invention provides an improved biomarker. In the context of the invention, the biomarker is a feature of a medical image that may be related to a medical condition and might therefore be referred to as an imaging biomarker. The biomarker may be employed as a diagnostic indicator or a prognostic indicator, for example by comparing the biomarker with a predetermined threshold. The biomarker can be used as a diagnostic indicator to diagnose a condition of a patient or as a prognostic indicator for a predictive assessment of a condition of a patient. Indeed, according to a third aspect of the invention there is provided a method of diagnosing or predicting a condition of a patient, comprising comparing a biomarker with a threshold, wherein the biomarker comprises a ratio of texture parameters determined from a medical image. According to a fourth aspect of the invention there is provided an apparatus for diagnosing or predicting a condition of a patient, comprising means for comparing a biomarker with a threshold, wherein the biomarker comprises a ratio of texture parameters determined from a medical image.

The biomarker has application in, particularly but not exclusively, the evaluation of cancer images, and in particular can be used for predictive assessment. Such a biomarker obtained from analysing images of an organ can be indicative of advanced disease and predictive of poor survival in patients. For-example, when obtained from a visually normal (apparently disease free) CT image of a liver, the biomarker can be predictive of patho-physiology, disease extent (or metastases) and poor survival of a patient following resection of colorectal cancer. Consequently, a modified surveillance strategy may be adopted for such patients. As another example, when obtained from a mammographic image (e.g. digitized mammography films), the biomarker can be indicative of cancer invasion and receptor status within mammographic abnormalities. As another example, when obtained from a CT image of the lungs, the biomarker can be indicative of the grading or staging of lung nodules and predictive of tumour metabolism in lung carcinoma. As another example, when obtained from a CT image of an oesophagus, the biomarker can be indicative of the extent, spread, grading or staging of oesophageal carcinoma and predictive of tumour metabolism. As another example, when obtained from a CT image of the mouth (e.g. a dental CT image) or a dental radiographic image (e.g. a digitised dental radiographic image), the biomarker can be indicative of extent, spread, grading or staging of dental carcinoma.

The biomarker also has application in the evaluation of images for a variety of other medical conditions not related to cancer. For example, when obtained from an MRI image of the brain, the biomarker can be indicative of schizophrenia and/or other brain disorders. As another example, when obtained from a CT image of the lungs, the biomarker can be indicative of pulmonary disorders.

The biomarker can be obtained by analysing conventional images, and therefore the invention can be easily implemented as an addition to existing image systems. Optionally, the image data may represent one of an X-ray image, in particular a tomography image (e.g. a hepatic, lung oesophagus or dental tomography image) or a mammography image, a magnetic resonance image (e.g. a brain image) and an ultrasound image. A tomography image may be, for example, a computed tomography (CT) image, which is also known as a computed axial tomography (CAT) image, or a positron emission tomography (PET) image or a single photon emission computed tomography (SPECT) image. The image is usually two-dimensional (e.g. an image slice), but may alternatively be threedimensional (e.g. an image volume).

The band-pass filters may differ in only bandwidth and be otherwise identical. In other words, the data may be filtered more than once with the same filter tuned to different bandwidths. The filtering is described as being performed with a plurality of filters having different bandwidths for clarity and conciseness. Optionally, the band-pass filters may be Laplacian of Gaussian (LoG) band-pass filters. Such a filter is advantageous in that it can be tuned easily to provide different bandwidths.

Optionally, the texture parameter may comprise an indicator of at least one of: mean gray-level intensity; entropy; uniformity.

Use of the terms "means for filtering", "means for determining" and such like is intended to be general rather than specific. The invention may be implemented using such separate components. However, it may equally be implemented using a single component such as an individual processor, digital signal processor (DSP) or central processing unit (CPU). Similarly, the invention could be implemented using a hard-wired circuit or circuits, such as an application-specific integrated circuit (ASIC), or by embedded software. Indeed, it can also be appreciated that the invention can be implemented using computer program code. According to a further aspect of the present invention, there is therefore provided computer software or computer program code adapted to carry out the method described above when processed by a processing means. The computer software or computer program code can be carried by a computer readable medium. The medium may be a physical storage medium such as a Read Only Memory (ROM) chip. Alternatively, it may be a disk such as a Digital Versatile Disk (DVD-ROM) or Compact Disk (CD-ROM). It could also be a signal such as an electronic signal over wires, an optical signal or a radio signal such as to a satellite or the like. The invention also extends to a processor running the software or code, e.g. a computer configured to carry out the method described above.

BRIEF DESCRIPTION OF DRAWINGS

The invention will now be described, by way of example only, with reference to the accompanying drawings wherein:

Figure 1 is a flow chart of a method of analysing medical image data in accordance with the invention;

Figure 2 is a block schematic diagram of an apparatus for analysing medical image data in accordance with the invention;

Figure 3 illustrates spatial and frequency domain representations of a LoG filter;

Figure 4 illustrates a frequency domain representation of a three-dimensional LoG filter;

Figure 5 is a table showing the relationship between standard deviation and filter width;

Figure 6 illustrates a hepatic computed tomography image of a liver, filtered with three different bandwidth filters, providing fine, medium and coarse filtering;

Figure 7 is a table of texture parameter values, and ratios of texture parameter values, of un-enhanced CT images of colorectal cancer patients;

Figure 8 is a table of mean grey-level texture parameter values, and ratios of texture parameter values, of contrast enhanced CT images of colorectal cancer patients;

Figure 9 is a graph illustrating a Kaplan-Meier survival curve for patients with normal liver appearances on conventional CT but liver relative texture (normalised coarse mean grey-level intensity) values above and below a threshold value of 1.13;

Figure 10 is graph illustrating the correlation of normalised coarse mean grey-level intensity with hepatic phosphorylation fraction index (HPFI) for patients with no liver metastases;

Figure 11 is a graph illustrating a Kaplan-Meier survival curve for patients with normal liver appearances on conventional CT but standardised uptake value of glucose (SUV) above and below a threshold value of 1.875;

Figure 12 is a graph illustrating the relationship between relative texture (ratio of fine to medium mean grey-level intensity) values and degree of invasion for breast cancer patients;

Figure 13 is a graph illustrating the relationship between relative texture (ratio of fine to coarse mean grey-level intensity) values and estrogen receptor status (ER);

Figure 14 is a graph illustrating the relationship between relative texture (ratio of medium to coarse mean grey-level intensity) values and progesterone receptor status (PR);

Figure 15 is a graph illustrating the relationship between relative texture (normalised coarse uniformity) values and tumour stages for non-small cell lung carcinoma patients;

Figure 16 is a graph illustrating the relationship between standardised uptake value of glucose (SUV) values and tumour stages for non-small cell lung carcinoma patients;

Figure 17 is graph illustrating the correlation of normalised coarse entropy with standardised uptake value of glucose (SUV) for non-small cell lung carcinoma patients; and

Figure 18 is a box and whisper plot of entropy calculated from medium to coarse texture ratio of three-dimensional whole brain grey matter CT images of schizophrenic patients and a control group of patients.

## DETAILED DESCRIPTION OF PREFERRED EMBODIMENTS

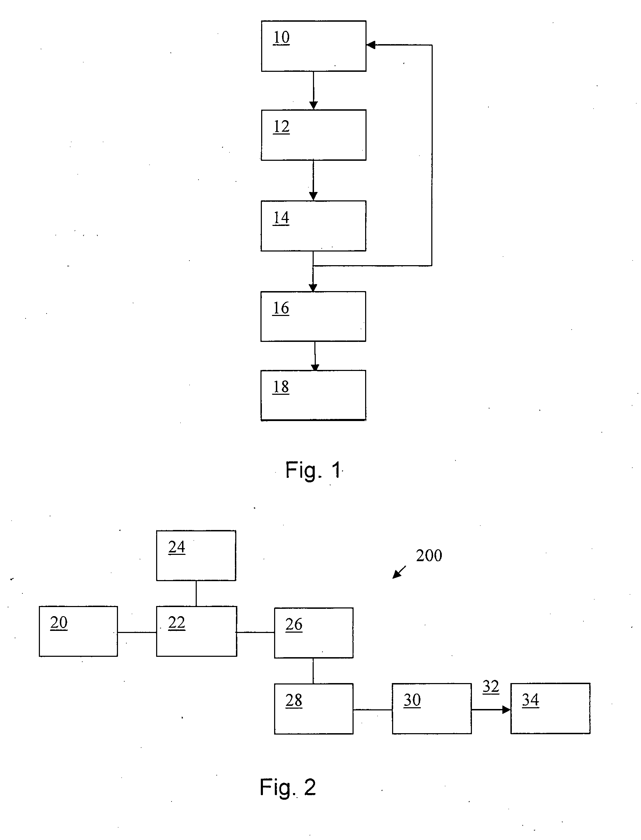

Referring to Figure 1, the method of analysing medical image data commences at step 10 by selecting the bandwidth of a filter. At step 12 the image data is filtered by the filter employing the selected bandwidth. At step 14 a texture parameter is determined from the filtered data. Flow then returns to step 10 where a different bandwidth is selected and then at step 12 the image data is filtered by the filter employing the different bandwidth, and then at step 14 a texture parameter is determined from the data filtered using different bandwidth. Steps 10, 12 and 14 may be repeated any desired number of times. For example, three different bandwidths may be used to provide fine, medium and coarse filtering and corresponding fine, medium and coarse texture parameters. At step 16 a ratio is calculated of two of the texture parameters corresponding to different filter bandwidths, and optionally additional ratios may be calculated using different pairs of the texture parameters. The ratio, or ratios, of texture parameters are delivered for use as a biomarker. At optional step 18, the biomarker may be compared with a predetermined threshold value and an indication generated according to whether the value of the biomarker is above or below the predetermined threshold value. A suitable threshold value can be determined by analysing patient data.

Although Figure 1 illustrates the image data being filtered using different bandwidths sequentially, the filtering using different bandwidths may alternatively be performed in parallel. In the specification and claims, the expression "plurality of band-pass filters each having a different bandwidth" is intended to encompass both fixed-bandwidth filters and a variable bandwidth filter, a different bandwidth being regarded as providing a different filter.

Referring to Figure 2, the apparatus for analysing medical image data comprises a data store 20 for storing the image data. An output of the data store 20 is coupled to an input of a filter 22 for filtering the image data. A further input of the filter 22 is coupled to a bandwidth controller 24. The bandwidth of the filter 22 is adaptable under the control of the bandwidth controller 24, thereby enabling the image data to be filtered using a plurality of different bandwidths. An output of the filter 22 is coupled to an input of a texture parameter determining stage 26, which for example may be implemented in a processor. For each bandwidth used by the filter 22 for filtering the image data, the texture parameter determining stage 26 determines a texture parameter from the filtered image data and stores the resulting texture parameter in parameter store 28. A ratio calculator 30 is coupled to the parameter store 28 and is adapted to calculate the ratio of two of the stored texture parameters corresponding to different filter bandwidths, and optionally to calculate additional ratios using different pairs of the stored texture parameters. The ratio calculator 30 provides on an output 32 the ratio, or ratios, of the texture parameters for use as a biomarker. Optionally the output of the ratio calculator 30 may be coupled to a comparator 34 which is adapted to compare the value of the biomarker with a predetermined threshold, and to generate an indication according to whether the value of the biomarker is above or below the predetermined threshold. A suitable threshold value can be determined by analysing patient data.

Although the apparatus illustrated in Figure 2 comprises a single filter which is adapted to filter the image data using different bandwidths sequentially, alternatively a plurality of filters may be used and may operate in parallel, each having a fixed bandwidth. In the specification and claims, the expression "plurality of band-pass filters each having a different bandwidth" is intended to encompass both fixed-bandwidth filters and a variable bandwidth filter, a different bandwidth being regarded as providing a different filter.

The method steps and the apparatus will now be described in more detail for the case of three different filter bandwidths corresponding to fine, medium and coarse texture parameters.

One type of filter that may be used for filtering the image data is a Laplacian of Gaussian (LoG) band-pass filter. This is a non-orthogonal Wavelet transform. This type of filter can be readily tuned so as to selectively extract scale based individual textures such as fine, medium and coarse texture. Wavelet transforms also tend to perform better than frequency domain based Fourier transforms, which lack spatial localisation. The two-dimensional (2-D) Gaussian distribution (G) is given by:

$$G(x, y) = e^{\frac{x^2 + y^2}{2\pi\sigma^2}} \tag{1}$$

where (x, y) are the spatial coordinates of the image matrix and sigma, $\sigma$, is the standard deviation.

The three-dimensional (3-D) Gaussian distribution (G) is given by

$$G(x, y, z) = e^{\frac{x^2 + y^2 + z^2}{2\pi\sigma^2}} \tag{2}$$

The Gaussian distribution effectively blurs the image, wiping out all structures at scales much smaller than the sigma value of the Gaussian distribution. This distribution has the desirable characteristics of being smooth and localised in both the spatial and frequency domains and is therefore less likely to introduce any changes that were not present in the original image. Thus, the Gaussian distribution enables the highlighting of only features of a particular size in images corresponding to a particular value.

One reason for using the Laplacian ($\nabla^2$) is that it is the lowest-order orientation-independent (isotropic) differential operator which inherently has less computational burden and can be used to detect intensity changes in an image that correspond to the zero-crossings of the filter. $\nabla^2 G$ is the Laplacian of Gaussian (LoG) filter, a circularly symmetric mexican-hat-shaped filter whose distribution in the 2-D and 3-D spatial domains are given by

$$\nabla^2 G(x, y) = \frac{-1}{\pi\sigma^4}\left(1 - \frac{x^2 + y^2}{2\sigma^2}\right)e^{-\left(\frac{x^2 + y^2}{2\sigma^2}\right)} \tag{3}$$

$$\nabla^2 G(x, y, z) = \frac{-1}{\pi\sigma^4}\left(1 - \frac{x^2 + y^2 + z^2}{2\sigma^2}\right)e^{-\left(\frac{x^2 + y^2 + z^2}{2\sigma^2}\right)} \tag{4}$$

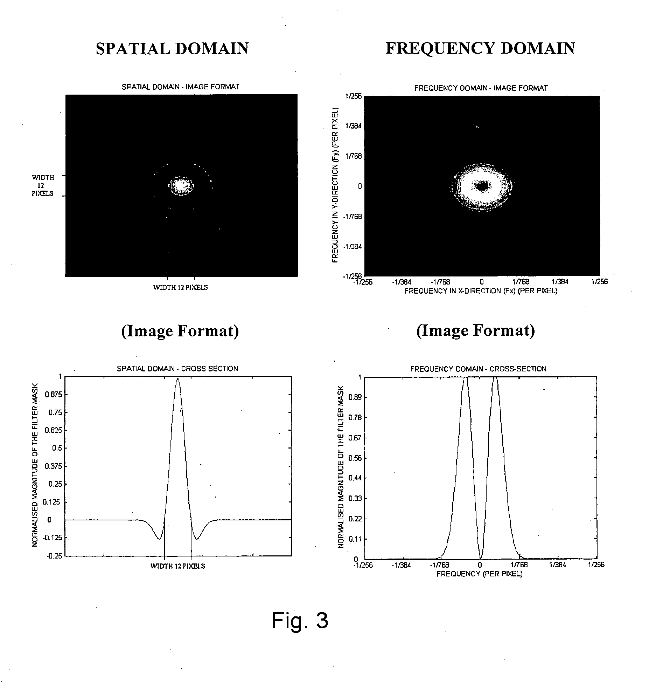

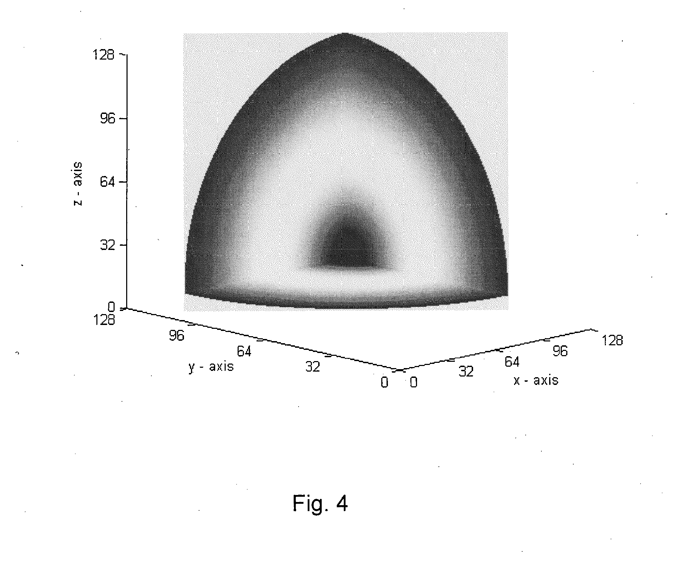

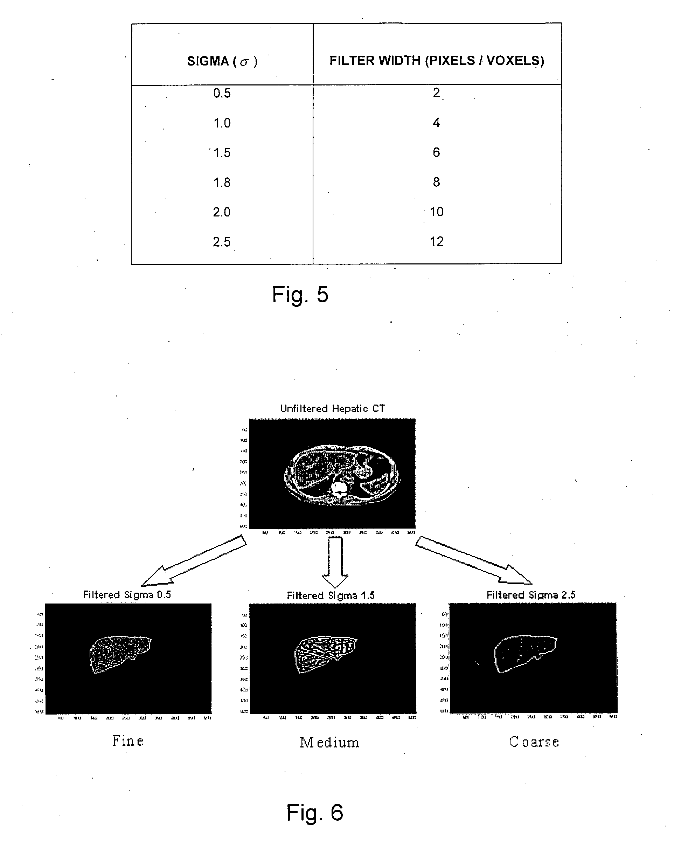

Figure 3 illustrates two-dimensional spatial and frequency domain representations of a LoG filter in the special and frequency domain at a standard deviation ($\sigma$) value of 2.5. Figure 4 is a frequency domain representation of the sub-volume of the absolute values of a three-dimensional LoG filter at a $\sigma$ value of 1.5. From the mathematical expression of this circularly symmetric filter at different values, the number of pixels/voxels representing the width between the diametrically opposite zero-crossing points in this filter can be calculated. The width of the filter at different $\sigma$ values is obtained by evaluating the LoG spatial distribution along the x and y directions. The width can be considered as the size at which the structures in the image will be highlighted and enhanced, whilst structures below this size will become blurred. The lower the $\sigma$ value, the smaller is the width of the filter in the spatial domain and the larger is the pass-band region of the filter in the frequency domain, highlighting fine details or features in the filtered image in the spatial domain. Similarly, the higher the $\sigma$ value, the higher is the width of the filter in the spatial domain; this corresponds to a smaller pass-band region of the filter in the frequency domain, highlighting coarse features in the filtered image in the spatial domain. The table of Figure 5 indicates the filter width in pixels corresponding to several $\sigma$ values.

Other types of band-pass filter characteristic may be used instead of LoG, for example Difference of Gaussian (DoG).

In hepatic CT images, fine texture may predominantly highlight hepatic parenchyma while medium to coarse texture may highlight blood vessels of varying size or hepatic tissue response. In mammography images fine texture may predominantly highlighf micro-calcification while medium to coarse texture may highlight calcification clusters. In a three dimensional MRI image of the brain or brain volume, fine texture may reflect thinner sensory areas within the cortex, medium texture may correspond to fundi of sulci and/or less prominent crowns of gyri, whiles coarse texture may correspond to prominent crowns of gyri. Figure 6 illustrates a hepatic computed tomography image of a liver, filtered with three different bandwidth filters, providing fine, medium and coarse filtering.

Filtration can be done in the spatial or frequency domain. In the spatial domain, the filter mask is convolved with the image, which involves intensive computation. It is more efficient to employ the filter in the frequency domain, as convolution of the filter mask and the image in the spatial domain is equivalent to multiplication of the Fourier transforms of the filter mask and the image in the frequency domain. The inverse Fourier transform of the filtered spectrum gives the resultant filtered image in the spatial domain. Also the accuracy of this filtration operation is improved when employed in the frequency domain, as the quantisation errors arising from the convolution of the filter, especially for small $\sigma$ values in the spatial domain, would distort the image.

The texture parameter may be determined from the filtered data using a mathematical descriptor such as Mean Grey-Level Intensity, which is an indicator of average tissue brightness, Entropy (e), which is an indicator brightness and inhomogeneity (irregularity), and Uniformity (u), which is an indicator of how close the image is to a uniform distribution of grey-levels. Entropy and Uniformity are image parameters that describe the distribution of tissue attenuation and represent texture that is generally not visually perceptible.

These texture parameters are defined mathematically as follows:

$$Mean\ Grey-Level\ Intensity\ (m) = \frac{1}{N}\sum_{(x,y)\in R}[a(x,y)] \tag{5}$$

$$Entropy(e) = -\sum_{l=1}^{k}[p(l)]\log_2[p(l)] \tag{6}$$

$$Uniformity(u) = \sum_{l=1}^{k}[p(l)]^2 \tag{7}$$

where R is the region of interest within the image, N is the total number of pixels in the region of interest R, $l$ is the number of grey-levels (for example $l$ = 1 to k indicates grey-level from 1 to k) in the region of interest R, and $p(l)$ the probability of the occurrence of the grey-level $l$ based on the image histograming technique:

The ratio of the texture parameters resulting from the use of different filter widths, for example fine to medium, fine to coarse and medium to coarse may be determined. Fine to medium texture ratio is calculated using the mathematical expressions (8) to (10) below.

$$\text{Fine to medium texture ratio for mean grey} - \text{level intensity} = \frac{\frac{1}{N}\sum_{(x,y)\in R}[a(x,y)_{\sigma=0.5}]}{\frac{1}{N}\sum_{(x,y)\in R}[a(x,y)_{\sigma=1.5}]} \tag{8}$$

$$\text{Fine to medium texture ratio for entropy} = \frac{-\sum_{l=1}^{k}[p(l)_{\sigma=0.5}]\log_2[p(l)_{\sigma=0.5}]}{-\sum_{l=1}^{k}[p(l)_{\sigma=1.5}]\log_2[p(l)_{\sigma=1.5}]} \tag{9}$$

$$\text{Fine to medium texture ratio for uniformity} = \frac{\sum_{l=1}^{k}[p(l)_{\sigma=0.5}]^2}{\sum_{l=1}^{k}[p(l)_{\sigma=1.5}]^2} \tag{10}$$

Furthermore, the ratios of texture parameters may be normalised with respect to the largest observed texture feature which corresponds to a filter $\sigma$ value of 2.5. Some examples of normalised ratios of texture parameters for $\sigma$ = 0.5 (normalised fine or fine to coarse ratio) and for $\sigma$ =1.5 (normalised medium or medium to coarse ratio) are defined below in expressions (11) to (16).

Fine to coarse texture ratio (Normalised fine)

$$\text{for mean grey} - \text{level intensity} = \frac{\frac{1}{N}\sum_{(x,y)\in R}[a(x,y)_{\sigma=0.5}]}{\frac{1}{N}\sum_{(x,y)\in R}[a(x,y)_{\sigma=2.5}]} \tag{11}$$

$$\text{Fine to coarse texture (Normalised fine) ratio for entropy} = \frac{-\sum_{l=1}^{k}[p(l)_{\sigma=0.5}]\log_2[p(l)_{\sigma=0.5}]}{-\sum_{l=1}^{k}[p(l)_{\sigma=2.5}]\log_2[p(l)_{\sigma=2.5}]} \tag{12}$$

$$\textit{Fine to coarse texture ratio (Normalised fine) for uniformity} = \frac{\sum\limits_{l=1}^{k}[p(l)_{\sigma=0.5}]^2}{\sum\limits_{l=1}^{k}[p(l)_{\sigma=2.5}]^2} \qquad (13)$$

*Medium to coarse texture ratio (Normalised medium)*

$$\textit{for mean grey} - \textit{level intensity} = \frac{\dfrac{1}{N}\sum\limits_{(x,y)\in R}[a(x,y)_{\sigma=1.5}]}{\dfrac{1}{N}\sum\limits_{(x,y)\in R}[a(x,y)_{\sigma=2.5}]} \qquad (14)$$

Medium to coarse texture ratio (Normalised medium)

$$\textit{for entropy} = \frac{-\sum\limits_{l=1}^{k}[p(l)_{\sigma=1.5}]\log_2[p(l)_{\sigma=1.5}]}{-\sum\limits_{l=1}^{k}[p(l)_{\sigma=2.5}]\log_2[p(l)_{\sigma=2.5}]} \qquad (15)$$

$$\textit{Medium to coarse texture ratio (Normalised medium) for uniformity} = \frac{\sum\limits_{l=1}^{k}[p(l)_{\sigma=1.5}]^2}{\sum\limits_{l=1}^{k}[p(l)_{\sigma=2.5}]^2} \qquad (16)$$

The use of a normalised texture ratio minimises the effects of any variations in CT attenuation values occurring from one patient to another and also reduces the effect of noise on texture quantification.

Each ratio of texture parameters, either normalised or non-normalised, can be used as a diagnostic indicator.

Some results of a study applying the method according to the invention to image data obtained from, firstly, colorectal cancer patients and, secondly, breast cancer patients are presented below to illustrate the effectiveness of the method.

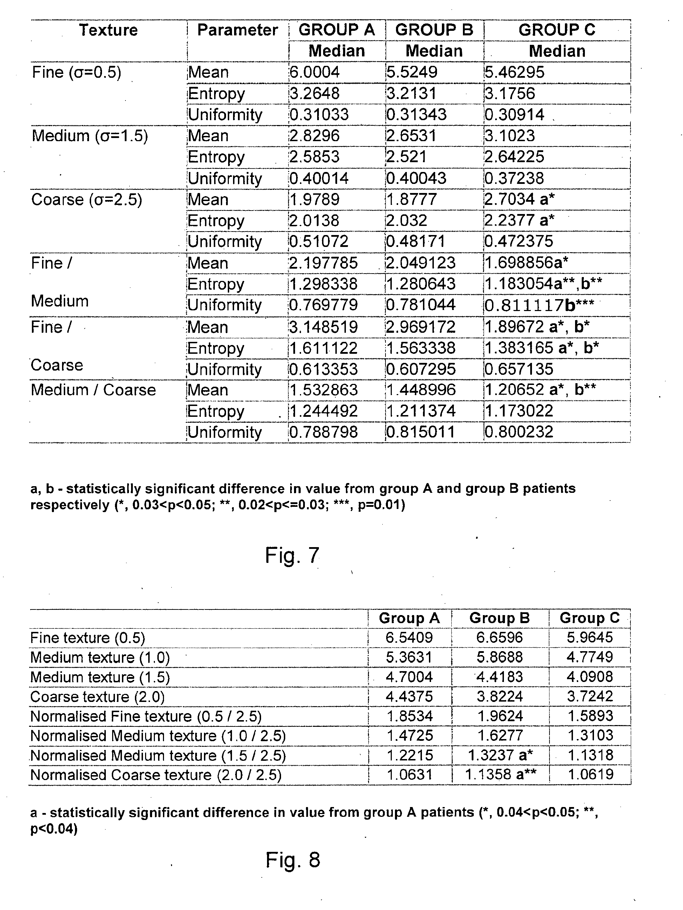

For the study of colorectal cancer, data obtained from three patient groups was compared; group A is 15 patients with no tumour, group B is 9 patients without liver metastases, and group C is 8 patients with liver metastases. Figure 7 is a table of texture parameter values and ratios of texture parameter values of unenhanced CT images of the three groups of patients, from which it can be seen that there is no significant difference between any texture parameter for groups A and B, although for coarse and medium texture images, there is a trend towards higher values for mean grey-level intensity and entropy in group C (liver metastases) as compared to groups A and B, reaching statistical significance for coarse texture images ($p < 0.05$, where p is a probability value indicative of statistical significance, and where low values of p indicate a high statistical significance). Greater differentiation of the patient groups was achieved by using ratios of texture parameter values. In particular, fine to medium texture parameter ratios were most significant in differentiating the different diagnostic groups. Comparing groups A and C, the most significant difference was obtained for the ratio of fine to medium texture using the texture parameter entropy ($p = 0.0257$) whilst the difference in this ratio for mean grey-level intensity was less significant (p = 0.049). For groups B and C, the most significant difference was obtained using the fine to medium texture ratio for the texture parameter uniformity (p = 0.0143). Entropy also discriminated these two groups, with the highest significance obtained using the fine to medium texture ratio (p = 0.03).

Figure 8 is a table of mean grey-level texture parameter values and ratios of texture parameter values of contrast-enhanced portal phase CT images of the three groups of patients. A typical contrast agent for CT is an iodine based compound. The invention can be applied also to images obtained for other temporal phases. From Figure 7 it can be seen that liver texture is significantly different in patients with extra-hepatic metastases (Group B) compared to patients with no tumour (Group A) as indicated by higher intensity values on normalised coarse texture (ratio for $\sigma$ = 2.0 and 2.5, p < 0.04) and normalised medium texture images (ratio for $\sigma$ = 1.5 and 2.5, 0.04 < p < 0.05).

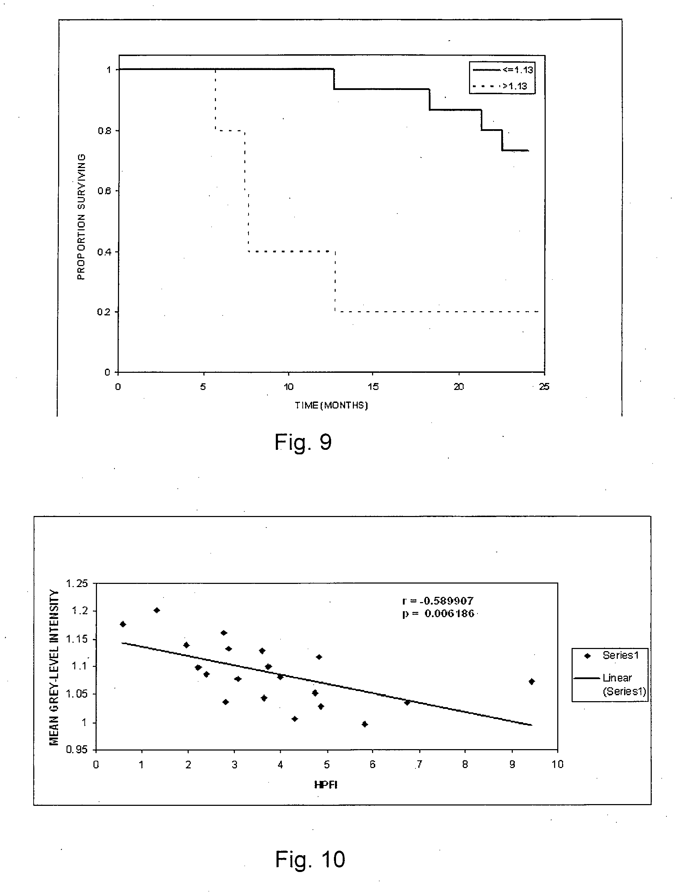

A normalised mean grey-level coarse texture parameter value greater than 1.13 indicates a likelihood of extra-hepatic metastases five times higher than for patients with lower texture parameter values with a sensitivity of 62.5% and specificity of 100% (p = 0.0035). Kaplan-Meier survival curves for patients with normal hepatic appearances on conventional CT separated by a normalised coarse texture were significantly different (p = 0.0074). Reduced survival was found for patients with liver texture values above 1.13. Therefore, a suitable value for the threshold value referred to above is 1.13. Figure 9 is a graph illustrating a Kaplan-Meier survival curve for patients with a normal liver appearance on conventional CT, with a normalised mean grey-level coarse liver texture parameter value greater than a threshold of 1.13 (broken line) and below 1.13 (solid line).

Furthermore, two related biological correlates for liver texture on portal phase CT in colorectal cancer patients without hepatic metastases were identified as hepatic blood flow and glucose metabolism. The hepatic phosphorylation fraction index (HPFI) of glucose which is derived from the ratio of standardised uptake value of glucose (SUV) in the liver obtained from PET and Total Hepatic Perfusion (THP) - combination of arterial (HAP) and portal perfusion (HPP) obtained from perfusion CT was identified as the most possible biological correlate for normalised mean coarse texture (r = -0.59, where r is the correlation, p = 0.0062, as indicated by Figure 10). This texture parameter also correlated inversely with hepatic glucose utilization (SUV: r = -0.587, p = 0.007) and positively with hepatic blood flow (THP: r = 0.512, p = 0.021 and HPP: r = 0.451, p = 0.046). A statistically significant positive correlation was also observed for normalised coarse uniformity parameter (HPFI: r = 0.552, p = 0.012 and SUV: r = 0.468, p = 0.038).

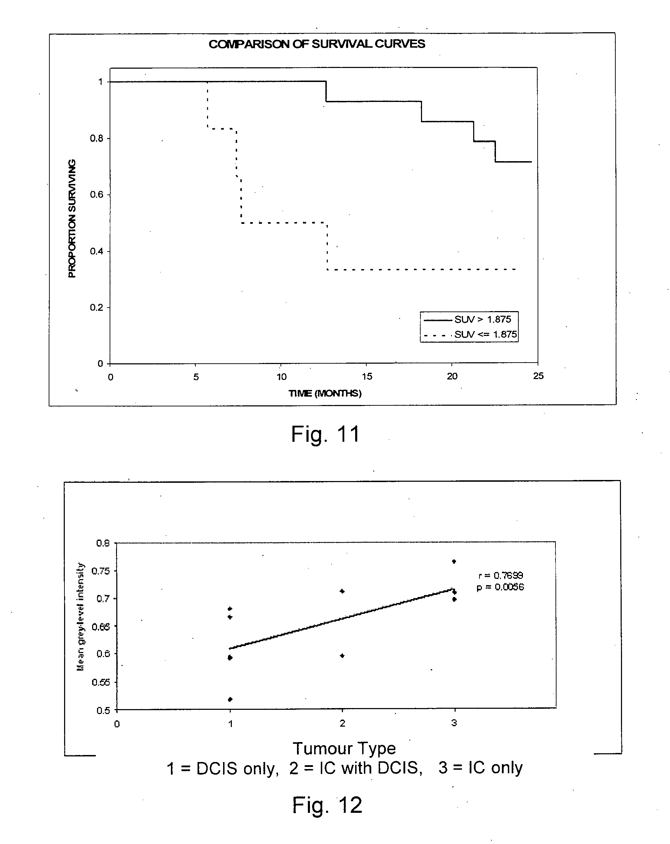

For comparison with Figure 9, Figure 11 illustrates the corresponding survival curves when hepatic glucose utilisation (p=0.045) was used as an indicator, with a hepatic SUV above (solid line) and below (broken line) a threshold value of 1.875. The survival curves in Figure 11 show less separation than the curves of Figure 9.

In the study of breast cancer, a significant relationship was observed between the fine/medium ratio of texture parameters (for $\sigma$ =0.5 and 1.5) and degree of invasion when considering only oblique and lateral projections, as illustrated in Figure 12 for DCIS only, DCIS and IC (Invasive carcinoma) only, and IC only patients.

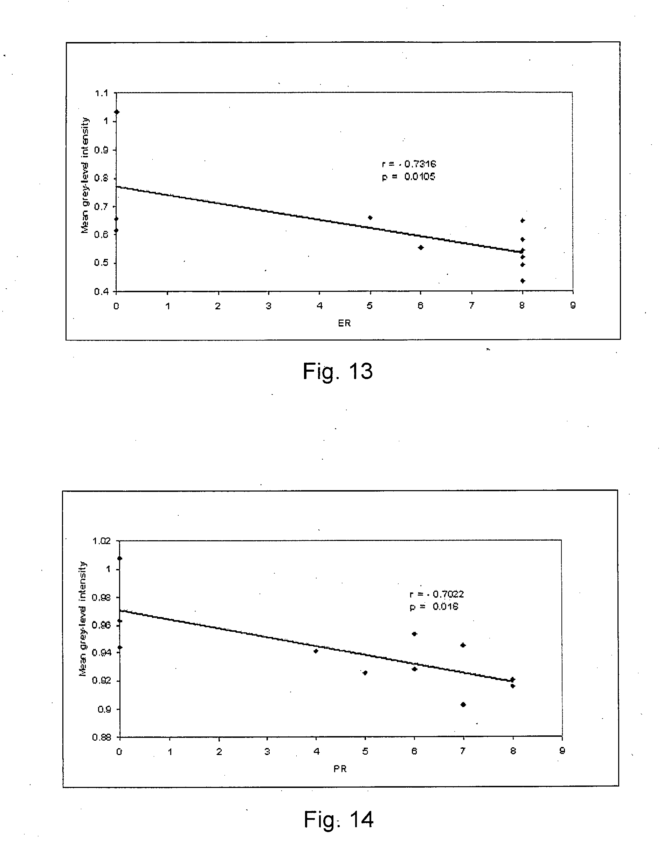

Furthermore, two biological correlates for mammographic texture for breast cancer patients were identified as estrogen receptor status and progesterone receptor status, thereby providing a "texture-molecular" relationship. Fine to coarse texture ratio (ratio for $\sigma$ = 0.5 and 2.5) showed the most significant inverse correlation with estrogen receptor (ER) status (r = -0.7316, p = 0.0105) as illustrated in Figure 13. Medium to coarse texture ratio (ratio for $\sigma$ = 2.0 and 2.5) showed significant inverse correlation with progesterone receptor (PR) status (r = -0.7022, p = 0.016) as illustrated in Figure 14.

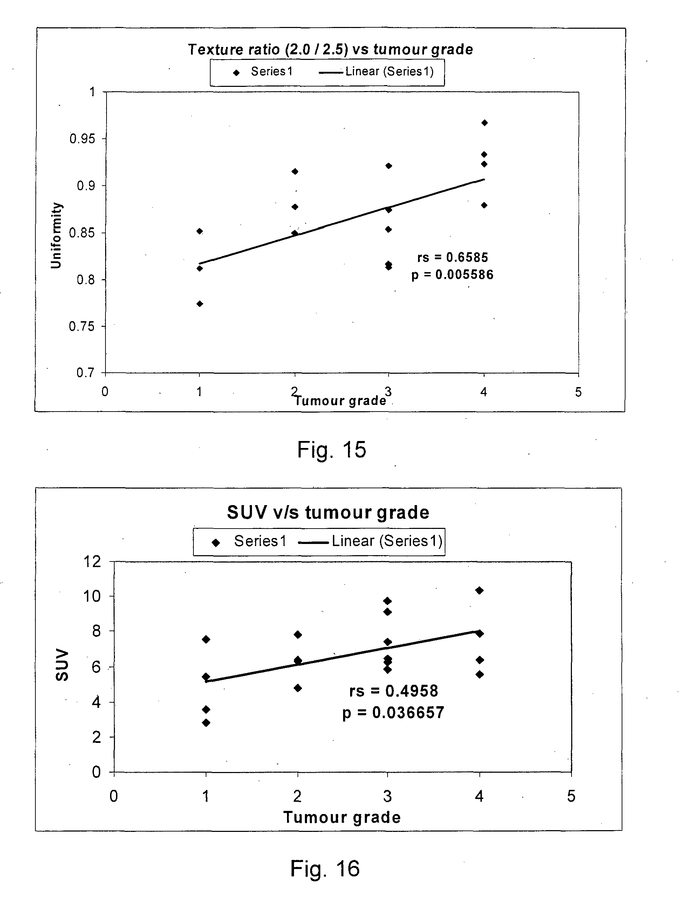

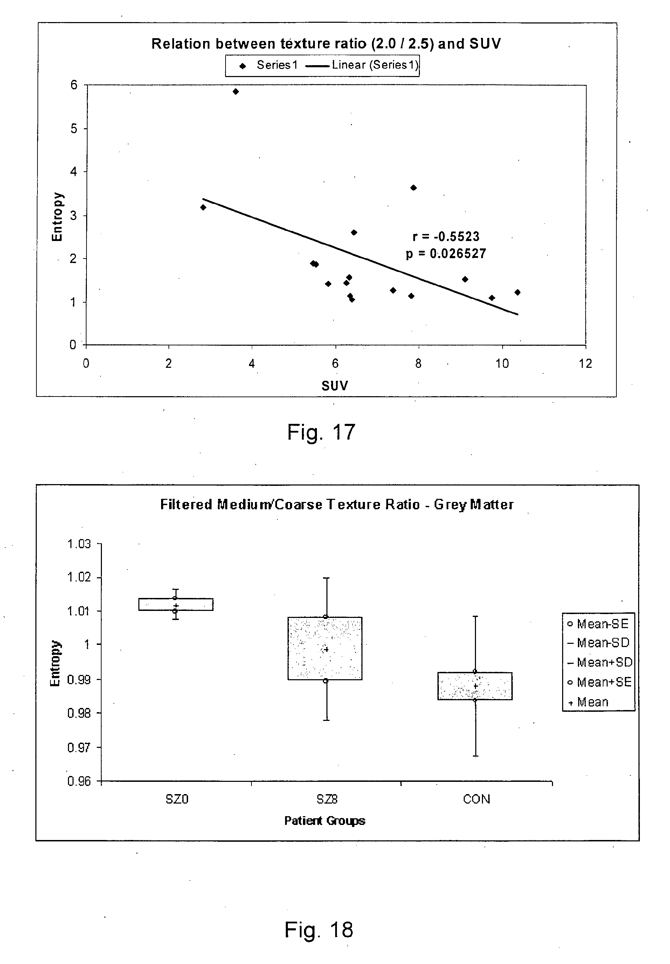

In the study of lung cancer (non-small cell lung carcinoma), a significant relationship was observed between texture ratios (ratio for $\sigma$ = 1.5 and 2.5, $\sigma$ = 1.8 and 2.5 and $\sigma$ = 2.0 and 2.5) within lung nodule on CT and tumour stage, with normalised coarse texture quantified as uniformity showing greatest indicator of tumour stage (grading) as illustrated in Figure 15. Figure 16 illustrates corresponding tumour stage predictability by standardised uptake values of glucose (SUV) in the lung nodule obtained from PET. The tumour stage predictability or disease grading using imaging parameter was greater for normalised coarse uniformity texture than SUV. This normalised coarse texture also correlated inversely with hepatic glucose utilization (entropy v/s SUV: r = - 0.552, p = 0.027 - Figure 17; mean grey-level intensity v/s SUV: r = -0.512, p = 0.043).

In the study of schizophrenia, a significant relationship was observed between the medium/coarse ratio of texture parameters (for $\sigma$ =1.0 and 1.5) for three-dimensional MRI whole brain grey matter images in schizophrenic patients versus a control group (mean grey-level intensity, p = 0.0271; entropy, p = 0.0114; and uniformity, p = 0.03). It was also found that an entropy value greater than 0.9976 indicated a greater variation in the distribution of grey matter features and predicted patients with schizophrenia (area under the receiver operating characteristic - ROC curve = 0.783, p=0.003, sensitivity = 80%, specificity = 74%). Figure 18 shows entropy calculated from the medium to coarse texture ratio for patient groups SZ0 (schizophrenic with presence of PMC1 gene expression) SZ8 (schizophrenic without presence of PMC1 gene expression) and CON (a control group). The ratio of medium-to-coarse texture that distinguished the overall group of schizophrenic patients from controls robustly distinguished the SZ0 patient group from controls (mean grey-level intensity, p = 0.0099; entropy, p = 0.0069; and uniformity, p = 0.0357). Also the relative medium-to-coarse entropy value exceeding 1.0052, indicated a robust differentiation (greater variation in the distribution of grey matter features) of SZ0 patients from controls (area under the ROC curve = 0.896, p=0.0001, sensitivity = 100% and specificity = 83%)

Thus, as described above the biomarker, may be employed to diagnose or predict a condition of a patient and to determine an appropriate program of treatment or a surveillance strategy.

The method according to the invention may be implemented in software on a general purpose computer or on a special purpose computer or in special purpose hardware.

The biomarker may comprise a single ratio of texture parameters, or may comprise more than one such ratio in combination, each ratio employing different measures of texture parameter.

## Claims

1. A method of analysing medical image data to produce a biomarker, comprising:

   - filtering the data with a plurality of band-pass filters each having a different bandwidth;
   - determining a texture parameter from the filtered data from each filter;
   - determining at least one ratio of the texture parameters for use as a biomarker.

2. A method as claimed in claim 1, wherein the band-pass filters are Laplacian of Gaussian band-pass filters.

3. A method as claimed in claim 1, or 2, wherein the texture parameter comprises an indicator of at least one of: mean gray-level intensity; entropy; uniformity.

4. A method as claimed in claim 1, 2, or 3, wherein the image data represents one of: an X-ray image; a magnetic resonance image; an ultrasound image; a tomography image; a hepatic tomography image; a positron emission tomography image; a single photon emission computed tomography image; a mammographic image;.

5. A method as claimed in any one of claims 1 to 4, further comprising employing the biomarker as a diagnostic indicator or a prognostic indicator.

6. A method as claimed in claim 5, wherein employing the biomarker as a diagnostic indicator or a prognostic indicator comprises comparing the biomarker with a threshold.

7. A method of diagnosing or predicting a condition of a patient, comprising comparing a biomarker with a threshold, wherein the biomarker comprises a ratio of texture parameters determined from a medical image.

8. The method of any one of the preceding claims, wherein the image data represents either a two-dimensional or a three-dimensional image.

9. Apparatus for analysing medical image data to produce a biomarker, comprising:

   - means for filtering medical image data with a plurality of band-pass filters each having a different bandwidth;
   - means for determining a texture parameter from the filtered data from each filter;
   - means for determining at least one ratio of the texture parameters for use as a biomarker.

10. Apparatus as claimed in claim 9, wherein the band-pass filters are Laplacian of Gaussian band-pass filters.

11. Apparatus as claimed in claim 8, or 9, comprising means for determining the texture parameter as an indicator of at least one of: mean gray-level intensity; entropy; uniformity.

12. Apparatus as claimed in claim 9, 10 or 11, comprising means for comparing the biomarker with a threshold.

13. Computer program code adapted to carry out the method of any one of claims 1 to 8 when processed by a processing means.

14. A computer readable medium comprising a computer program adapted to perform the method of any one of claims 1 to 8.

15. Apparatus for diagnosing or predicting a condition of a patient, comprising means for comparing a biomarker with a threshold, wherein the biomarker comprises a ratio of texture parameters determined from a medical image.

```
        ┌─────────────┐
        │     10      │◄────────┐
        └─────────────┘         │
               │                │
               ▼                │
        ┌─────────────┐         │
        │     12      │         │
        └─────────────┘         │
               │                │
               ▼                │
        ┌─────────────┐         │
        │     14      │         │
        └─────────────┘         │
               │────────────────┘
               ▼
        ┌─────────────┐
        │     16      │
        └─────────────┘
               │
               ▼
        ┌─────────────┐
        │     18      │
        └─────────────┘
```

Fig. 1

```
              ┌─────────┐
              │   24    │
              └─────────┘
                   │
┌─────────┐   ┌─────────┐   ┌─────────┐            200
│   20    │───│   22    │───│   26    │           ↙
└─────────┘   └─────────┘   └─────────┘
                                 │
                            ┌─────────┐   ┌─────────┐  32  ┌─────────┐
                            │   28    │───│   30    │─────►│   34    │
                            └─────────┘   └─────────┘      └─────────┘
```

Fig. 2

## SPATIAL DOMAIN

FREQUENCY DOMAIN

**(Image Format)**

**(Image Format)**

# Fig. 3

Fig. 4

| SIGMA ($\sigma$) | FILTER WIDTH (PIXELS / VOXELS) |
|---|---|
| 0.5 | 2 |
| 1.0 | 4 |
| 1.5 | 6 |
| 1.8 | 8 |
| 2.0 | 10 |
| 2.5 | 12 |

Fig. 5

Unfiltered Hepatic CT

Filtered Sigma 0.5 — Fine

Filtered Sigma 1.5 — Medium

Filtered Sigma 2.5 — Coarse

Fig. 6

| Texture | Parameter | GROUP A Median | GROUP B Median | GROUP C Median |
|---|---|---|---|---|
| Fine (σ=0.5) | Mean | 6.0004 | 5.5249 | 5.46295 |
| | Entropy | 3.2648 | 3.2131 | 3.1756 |
| | Uniformity | 0.31033 | 0.31343 | 0.30914 |
| Medium (σ=1.5) | Mean | 2.8296 | 2.6531 | 3.1023 |
| | Entropy | 2.5853 | 2.521 | 2.64225 |
| | Uniformity | 0.40014 | 0.40043 | 0.37238 |
| Coarse (σ=2.5) | Mean | 1.9789 | 1.8777 | 2.7034 a* |
| | Entropy | 2.0138 | 2.032 | 2.2377 a* |
| | Uniformity | 0.51072 | 0.48171 | 0.472375 |
| Fine / Medium | Mean | 2.197785 | 2.049123 | 1.698856a* |
| | Entropy | 1.298338 | 1.280643 | 1.183054a**,b** |
| | Uniformity | 0.769779 | 0.781044 | 0.811117b*** |
| Fine / Coarse | Mean | 3.148519 | 2.969172 | 1.89672 a*, b* |
| | Entropy | 1.611122 | 1.563338 | 1.383165 a*, b* |
| | Uniformity | 0.613353 | 0.607295 | 0.657135 |
| Medium / Coarse | Mean | 1.532863 | 1.448996 | 1.20652 a*, b** |
| | Entropy | 1.244492 | 1.211374 | 1.173022 |
| | Uniformity | 0.788798 | 0.815011 | 0.800232 |

a, b - statistically significant difference in value from group A and group B patients respectively (*, 0.03<p<0.05; **, 0.02<p<=0.03; ***, p=0.01)

## Fig. 7

| | Group A | Group B | Group C |
|---|---|---|---|
| Fine texture (0.5) | 6.5409 | 6.6596 | 5.9645 |
| Medium texture (1.0) | 5.3631 | 5.8688 | 4.7749 |
| Medium texture (1.5) | 4.7004 | 4.4183 | 4.0908 |
| Coarse texture (2.0) | 4.4375 | 3.8224 | 3.7242 |
| Normalised Fine texture (0.5 / 2.5) | 1.8534 | 1.9624 | 1.5893 |
| Normalised Medium texture (1.0 / 2.5) | 1.4725 | 1.6277 | 1.3103 |
| Normalised Medium texture (1.5 / 2.5) | 1.2215 | 1.3237 a* | 1.1318 |
| Normalised Coarse texture (2.0 / 2.5) | 1.0631 | 1.1358 a** | 1.0619 |

a - statistically significant difference in value from group A patients (*, 0.04<p<0.05; **, p<0.04)

## Fig. 8

Fig. 9

Fig. 10

Fig. 11

Tumour Type

1 = DCIS only,  2 = IC with DCIS,   3 = IC only

Fig. 12

Fig. 13

Fig. 14

Fig. 15

Fig. 16

Fig. 17

Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **MALA K ; SADASIVAM V.** Automatic segmentation and classification of diffused liver diseases using wavelet based texture analysis and neural network. *INDICON, 2005 Annual IEEE 2005,* 2005, 216-219

- **MIR A.H. ; HANMANDLU M. ; TANDON S.N.** Texture analysis of CT-images for early detection of liver malignancy. *Biomed Sci Instrum.,* 1995, vol. 31, 213-7
- **BRAKE G.M. ; KARSSEMEIJER N. ; HENDRIKS J.H.** An automatic method to discriminate malignant masses from normal tissue in digital mammograms. *Phys. Med. Biol.,* 2000, vol. 45, 2843-2857